(19) Europäisches Patentamt
European Patent Office
Office européen des brevets

(11) EP 1 251 183 A2

(12) EUROPEAN PATENT APPLICATION

(43) Date of publication:
23.10.2002 Bulletin 2002/43

(51) Int Cl.[7]: C12Q 1/68

(21) Application number: 02388014.9

(22) Date of filing: 18.02.2002

(84) Designated Contracting States:
AT BE CH CY DE DK ES FI FR GB GR IE IT LI LU MC NL PT SE TR
Designated Extension States:
AL LT LV MK RO SI

(30) Priority: 18.04.2001 US 284729 P

(71) Applicant: Exiqon A/S
2950 Vedbaek (DK)

(72) Inventors:
- Jacobsen, Nana
2820 Gentofte (DK)
- Jakobsen, Mogens Havsteen
2720 Vanlose (DK)
- Skouv, Jan
3060 Espergaerde (DK)

(74) Representative: Rasmussen, Torben Ravn
Bygstubben 9
2950 Vedbaek (DK)

# (54) Improved helper probes for detection of a target sequence by a capture oligonucleotide

(57) A method for enhancing hybridisation of a capture oligonucleotide to a target sequence using a helper probe comprising modified nucleotide residues is disclosed. The method exhibits significantly improved binding abilities. In particular the method is suitable for detection of SNP sites.

EP 1 251 183 A2

## Description

### INTRODUCTION

The present invention relates to helper probes for enhancing the ability of a capture oligonucleotide to bind with high affinity and specificity to a target sequence, which may be located in a sequence having a complex secondary structure.

The capture oligonucleotide according to the present invention consists of an oligonucleotide that can exhibit significant hybridization properties, including significant discrimination between fully matched target oligonucleotides and oligonucleotides containing one or more mismatches.

Further, the invention relates to the use of oligonucleotides as helper probes for enhancement of the signal strength in an assay where a capture oligonucleotide is binding a target sequence.

Because of the high specificity that may be obtained by the method according to the invention it is particular suited for detection of single nucleotide polymorphism (SNP).

The invention relates also to a kit for use in diagnostic assays for detection of the presence of a target sequence in a given sample.

### BACKGROUND FOR THE INVENTION

In molecular biology, oligonucleotides are routinely used for a variety of purposes such as for example (i) as hybridization probes in the capture, identification and quantification of target nucleic acids (ii) as affinity probes in the purification of target nucleic acids (iii) as primers in sequencing reactions and target amplification processes such as the polymerase chain reaction (PCR) (iv) to clone and mutate nucleic acids and (vi) as building blocks in the assembly of macromolecular structures.

The fundamental property of oligonucleotides, however, which underlies all uses is their ability to recognize and hybridize sequence specifically to complementary single stranded nucleic acids employing either Watson-Crick hydrogen bonding (A-T and G-C) or other hydrogen bonding schemes such as the Hoogsteen mode.

Two important hybridization characteristics of a given oligonucleotide are affinity and specificity. Affinity is a measure of the binding strength of the oligonucleotide to its complementary target sequence (expressed as the thermostability ($T_m$) of the duplex). Specificity is a measure of the ability of the oligonucleotide to discriminate between a fully complementary and a mismatched target sequence. In other words, specificity is a measure of the loss of affinity associated with mismatched nucleobase pairs in the target.

In general, an increase in the affinity of an oligonucleotide occurs at the expense of specificity and vice-versa. This can pose problems with use of oligonucleotides. For instance, in diagnostic procedures, the oligonucleotide needs to have both high affinity to secure adequate sensitivity of the test and high specificity to avoid false positive results.

In situations requiring detection of single-base differences between like sequences (e.g., the wild type and a mutant form of a gene) , the parameters of the analysis require the highest level of resolution. For cases in which the position of the nucleotide in question is known in advance, several methods have been developed for examining single base changes without direct sequencing. For example, if a mutation of interest happens to fall within a restriction recognition sequence, a change in the pattern of digestion can be used as a diagnostic tool (e.g., restriction fragment length polymorphism [RFLP] analysis). In this way, single point mutations can be detected by the creation or destruction of RFLPs.

Single-base mutations have also been identified by cleavage of RNA-RNA or RNA-DNA heteroduplexes using Rnase H (Myers et al., Science 230:1242 [1985] and Winter et al., Proc. Natl. Acad. Sci. USA 82:7575 [1985]). Mutations are detected and localized by the presence and size of the RNA fragments generated by cleavage at the mismatches. Single nucleotide mismatches in DNA heteroduplexes are also recognized and cleaved by some chemicals, providing an alternative strategy to detect single base substitutions, generically named the "Mismatch Chemical Cleavage" (MCC) (Gogos et al., Nucl. Acids Res., 18:6807-6817 [1990]). However, this method requires the use of osmium tetroxide and piperidine, two highly noxious chemicals, which are not suited for use in a clinical laboratory. In addition, all of the mismatch cleavage methods lack sensitivity to some mismatch pairs, and all are prone to background cleavage at sites removed from the mismatch.

RFLP analysis suffers from low sensitivity and requires a large amount of sample. When RFLP analysis is used for the detection of point mutations, it is, by its nature, limited to the detection of only those single base changes which fall within a restriction sequence of a known restriction endonuclease. Moreover, the majority of the available enzymes have 4 to 6 base-pair recognition sequences, and cleave too frequently for many large-scale DNA manipulations (Eckstein and Lilley (eds.), Nucleic Acids and Molecular Biology, vol. 2, Springer-Verlag, Heidelberg [1988]). Thus, it is applicable only in a small fraction of cases, as most mutations do not fall within such sites.

EP 318 245 A2 discloses the use of helper oligonucleotides for enhancing nucleic acid hybridisations. Helper

oligonucleotides are selected to bind to the target nucleic acid sequence and impose a different secondary or tertiary structure on the target to facilitate the binding of the probe to the target. The helper oligonucleotide may be a relative short multimer of either RNA, DNA or analogues of phosphatediester backbone of DNA or RNA in their usual forms, where DNA is preferred.

It thus would be desirable to have new and improved helper oligonucleotides. It would be particularly desirable to have new helper oligonucleotides that exhibit enhanced specificity and affinity.

## SUMMARY OF THE INVENTION

The present invention relates to a novel method of improving nucleic acid hybridisation using helper probes comprising modified nucleic acid residues, such as LNA residues. In particular the invention pertains to a method for detection of a nucleotide target sequence in a sample by hybridisation using a hybridisation mixture comprising a capture oligonucleotide and a helper probe capable of enhancing the binding of said capture oligonucleotide to said nucleotide target sequence, wherein the helper probe is an oligonucleotide comprising modified nucleic acid residues.

In one aspect of the invention, the method uses oligonucleotides, comprising of monomers of a novel class of DNA analogues, designated locked nucleic acid (LNA). LNA oligonucleotides obey Watson-Crick base-pairing rules and form duplexes that are significantly more stable than similar duplexes formed by DNA oligonucleotides. In addition, LNA oligonucleotides are capable of hybridising with double-stranded DNA target molecules as well as RNA secondary structures by strand invasion.

The inventors have surprisingly recognized that oligonucleotides comprising monomers of modified nucleotides, such as LNA, provide for a significant larger enhancement when used as helper probes compared with DNA helper probes. In some occasions, the oligonucleotide comprising modified nucleotides is capable of making hybridisation events happening that would not have occurred in the absence of the modified oligonucleotide.

Preferred helper probes according to the invention are oligonucleotides containing a major portion (particularly greater than 50 percent of total oligonucleotide residues) of modified nucleic acid residues and a minor portion of non-modified nucleic acid residues. In one aspect of the invention it is preferred that the helper probe of the invention is fully modified, that is, all the nucleotides of the oligonucleotide are modified.

Also preferred in some aspects of the invention are oligonucleotide as helper probes that do not have greatly extended stretches of modified DNA or RNA residues, e.g. greater than about 4, 5 or 6 consecutive modified DNA or RNA residues. That is, preferably one or more non-modified DNA or RNA will be present after a consecutive stretch of about 3, 4, 5 or 6 modified nucleic acids. A gabmer, i.e. a modified oligonucleotide having flanking blocks of modified nucleic acids and a centre portion of non-modified (natural) DNA or RNA, may also be contemplated.

While oligonucleotides having such arrangement of modified nucleic acid residues are preferred, oligonucleotides having other arrangements of modified residues, particularly LNA residues, will also be useful in the methods of the invention.

A variety of modified nucleic acids may be employed in oligonucleotides of the invention. Generally preferred modified nucleic acids have the ability of increasing the affinity of the oligonucleotide to a hybridization partner. Generally, increased affinity can be determined by increased $T_m$. Specifically preferred modified nucleic acids for use as units of oligonucleotides of the invention include locked nucleic acids, (which include bicyclic and tricyclic DNA or RNA having a 2'-4' or 2'-3' sugar linkage); 2'-deoxy-2'-fluoro ribonucleotides; 2'-*O*-methyl ribonucleotides; 2'-*O*-methoxyethyl ribonucleotides; peptide nucleic acids; 5-propynyl pyrimidine ribonucleotides; 7-deazapurine ribonucleotides; 2,6-diaminopurine ribonucleotides; and 2-thio-pyrimidine ribonucleotides.

The invention also includes methods for use of the helper probes according to the invention, particularly in a variety of hybridization reactions, e.g. in connection with a capture probe in a SNP assay, in facilitating the attachment of a Taqman probe, a Molecular Beacon, and the like.

The invention further includes kits and methods for detection of single base pair changes between wild type genes and alleles and are especially useful in single nucleotide polymorphism (SNP) detection.

Kits suitable for assay systems are also provided which generally comprise an assay substrate platform packaged with or otherwise containing one or more capture oligonucleotides and one or more helper probes of the invention. The one or more capture oligonucleotides may be immobilized, e.g. by a covalent linkages, to the substrate surface. The assay may be used to conduct e.g. a diagnostic test, such as genotyping or detection of a disease marker from a fluid sample (e.g. patient's blood, urine or the like).

## SHORT DESCRIPTION OF THE DRAWINGS

Figure 1(A-D) are graphs showing the results of Example 1, particularly the use of LNA helper probes to improve the capture of single-stranded DNA targets by immobilized anthraquinone-coupled LNA capture probes

Figure 2 is a graph showing the results of Example 4, particularly the use of LNA helper probes for the specific

capture of PCR amplicons by immobilized anthraquinone-coupled LNA capture probes.

Figure 3 is a graph showing the results of Example 5, particularly the use of LNA helper probes for the specific capture of PCR amplicons based on patient samples.

## DETAILED DESCRIPTION OF THE INVENTION

The term "target" or "target sequence" is throughout this description intended to mean the nucleotide sequence that has to be detected in the method according to the invention. The target may for example comprise a single nucleotide polymorphism (SNP) site.

The term "capture oligonucleotide" is intended to mean an oligonucleotide that is capable of binding to the target by hybridisation. In case that the target comprises a SNP the capture oligonucleotide must be capable of discriminating between the different alleles of said site.

Capture oligonucleotides may in principle be made of any nucleotides or nucleotide analogues, which enable the resulting capture oligonucleotide to bind to the target sequence with a sufficient affinity and specificity. As examples of target nucleotides may be mentioned DNA, RNA, analogues such as PNA and LNA, and modifications thereof. The capture oligonucleotide may be homomeric i.e. comprising only one category of monomers, or it may be heteromeric i.e. comprising more that one category of monomers.

Capture oligonucleotides are in one aspect of the invention labelled in order to facilitate detection of capture oligonucleotides bound to the target.

The term "helper probe" is intended to mean an oligonucleotide that is capable of enhancing the binding of the capture oligonucleotide to the target.

Without wishing to be bound by theory it is believed that the helper probe is exerting its enhancing effect be reordering of breaking complex secondary structure at or around the target. It may be of advantage to increase the stringency, e.g. increasing the temperature or the ion strength, to facilitate the incorporation of the helper probe at or adjacent to the complex secondary structure.

The helper probe is also known as "helper oligonucleotide", "helper oligo", "enhancer element", or "enhancer". The person skilled in the art will appreciate that these terms may be regarded as equivalents.

The term "complex secondary structure" is to be understood as a secondary structure that is not purely linear but usually comprising single stranded stretches as well as double stranded stretches. Examples of complex secondary structure comprise double strands, stem and loop structures, and hairpins etc. tRNAs are other examples molecules having complex secondary structures.

The person skilled in the art will know how to select candidate sequences for the helper probes based on his skills and the sequence of the target and surroundings, i.e. the nucleotide sequences adjacent to the target as well as the complementary target sequence.

Candidate helper probe sequences may be selected so they are essentially complementary to the same strand as the capture oligonucleotide, or to a strand participating in secondary structures with the target strand.

In this connection the term "essentially complementary" is intended to mean capable of binding to said sequence under the condition in question.

The sequence of the helper probe is preferably selected so that the linear distance between the site where the helper probe binds and the target in question is in the range of 1-500 bases. The linear distance is understood as the distance along the nucleotide strand to which the capture oligonucleotide and the helper probe binds.

Alternatively, the sequence of the helper probe may be selected so that it binds to a sequence that participate in secondary structures affecting the target strand at a position within 500 bases from the target.

The unique properties of LNA allow for designing of specific LNA oligonucleotides for use as helper probes to improve nucleic acid hybridization by unwinding complex secondary structures in double-stranded DNA hybridization targets and RNA hybridization targets, as well as by suppressing competition from non-target sequences.

Similarly, LNA helper probes may be used in DNA sequencing aiming at improved throughput in large-scale, shotgun genome sequencing projects, improved throughput in capillary DNA sequencing (e.g. ABI prism 3700) as well as at an improved method for 1) sequencing large, tandemly repeated genomic regions, 2) closing gaps in genome sequencing projects and 3) sequencing of GC-rich templates. In DNA sequencing, oligonucleotide sequencing primers are combined with LNA helper probes for the read-through of GC-rich and/or tandemly repeated genomic regions, which often present many challenges for genome sequencing projects.

As discussed above, new chimeric oligonucleotides are provided that contain a mixture of non-modified nucleic acids and modified (non-natural) nucleic acids. In a particular aspect of the invention the chimeric oligonucleotide entirely consist of modified nucleic acid residues. In the following the term "oligonucleotides" will be discussed, and particular preferred oligonucleotides for use according to the invention will be discussed.

In the following discussion of oligonucleotides it is intended that the described oligonucleotide may be used as capture oligonucleotide or as helper probes, unless otherwise indicated. The use of this term is for convenience

only, to avoid repetition of the enumeration of the possible configurations for this method, and it is intended that each of the embodiments described below may be used in combination with any probe/target configurations (e.g., labelled probes and captured target DNA and vice versa).

Oligonucleotides of the invention preferably contain at least 50 percent or more, more preferably 55, 60, 65, or 70 percent or more of modified nucleic acid residues, based on total residues of the oligo. A non-modified nucleic acid as referred to herein means that the nucleic acid upon incorporation into a 10-mer oligomer will not increase the $T_m$ of the oligomer in excess of 1°C or 2°C. More preferably, the non-modified nucleic acid residue is a substantially or completely "natural" nucleic acid, i.e. containing a non-modified base of uracil, cytosine, thymine, adenine or guanine and a non-modified pentose sugar unit of β-D-ribose (in the case of RNA) or β-D-2-deoxyribose (in the case of DNA).

The capture oligonucleotide may also comprise one or more modified nucleic acid residues. The capture oligonucleotide of the invention suitably may contain only a single modified nucleic acid residue, but preferably an oligonucleotide will contain 2, 3, 4 or 5 or more modified nucleic acid residues. Typically preferred is where an oligonucleotide contains from about 5 to about 40 or 45 percent modified nucleic acid residues, based on total residues of the oligo, more preferably where the oligonucleotide contains from about 5 or 10 percent to about 20, 25, 30 or 35 percent modified nucleic acid residues, based on total residues of the oligo.

Preferred modified nucleic acids residues have the ability of increasing the affinity of the oligonucleotide to a hybridization partner. Generally, increased affinity can be determined by increased $T_m$. Preferably, a modified nucleic acid will increase the $T_m$ of 15-mer oligo by at least about 1°C or 2°C, more preferably at least about 3, 4 or 5°C.

The method of the invention may involve the use of primers during the preparation of the sample to be analysed. Especially, the sample may be amplicons from an amplification reaction, like PCR or NASBA. When primers are employed during the production of the sample to be analysed, such primers may comprise non-modified nucleic acid residues and a mixture of non-modified and modified nucleic acid residues. Particularly preferred oligonucleotides used as primers contain a non-modified DNA or RNA residue at the 3' and/or 5' ends and a modified nucleic acid residue at one position upstream from (generally referred to as the -1 position) either or both of the 3' or 5' terminal non-modified nucleic acid residues. More particular, preferred primers of the invention include those of the following formula I:

$$5'\text{-}X^1\text{-}X^2\text{-}(X^3)_n\text{-}X^4\text{-}X^5\text{-}3' \qquad \text{I}$$

wherein each of $X^1$, $X^2$, $X^3$, $X^4$ and $X^5$ are linked nucleic acid residues;
at least one of $X^2$ and $X^4$ is a modified nucleic acid residue; and n is an integer of 0 or greater. Preferably n is from 1 to about 50, more preferably n is 1, 2, 3, 4, 5, 6, 7, 8, 9, 10, 11, 12, 13, 14, 15, 20, 25 or 30. Each $X^3$ may be the same or different.

One or more of the primers can be conjugated to a reporter group to allow for an easy detection of the resulting amplicons.

LNA residues are in general particularly preferred modified nucleic acids for incorporation into an oligonucleotide of the invention. LNAs are described in WO 99/14226, which is incorporated herein by reference. Additionally, the nucleic acids may be modified at either the 3' and/or 5' end by any type of modification known in the art. For example, either or both ends may be capped with a protecting group, attached to a flexible linking group, attached to a reactive group to aid in attachment to the substrate surface, etc.

As disclosed in WO 99/14226, LNA are a novel class of DNA analogues that form DNA- or RNA-heteroduplexes with exceptionally high thermal stability. LNA monomers include bicyclic compounds as shown immediately below:

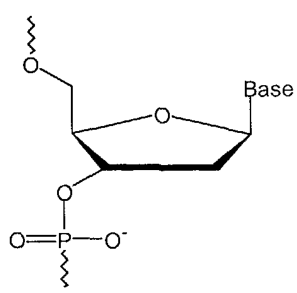

DNA

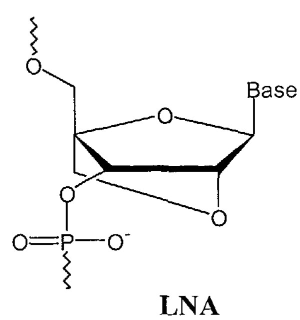

LNA

A reference herein to Locked Nucleoside Analogues, LNA or similar term is inclusive of such compounds as disclosed in WO 99/14226.

Preferred LNA monomers and oligomers can share chemical properties of DNA and RNA; they are water soluble, can be separated by agarose gel electrophoresis, can be ethanol precipitated, etc.

Introduction of LNA monomers into either DNA, RNA or pure LNA oligonucleotides results in extremely high thermal stability of duplexes with complimentary DNA or RNA, while at the same time obeying the Watson-Crick base pairing rules. In general, the thermal stability of heteroduplexes is increased 3-8°C per LNA monomer in the duplex. Oligonucleotides containing LNA can be designed to be substrates for polymerases (e.g. *Taq* polymerase), and PCR based on LNA primers is more discriminatory towards single base mutations in the template DNA compared to normal DNA-primers (i.e. allele specific PCR). Furthermore, very short LNA oligos (e.g. 8-mers), which have high $T_m$'s when compared to similar DNA oligos, can be used as highly specific catching probes with outstanding discriminatory power towards single base mutations (i.e. SNP detection).

Oligonucleotides containing LNA are readily synthesized by standard phosphoramidite chemistry. The flexibility of the phosphoramidite synthesis approach further facilitates the easy production of LNA oligos carrying all types of standard linkers, fluorophores and reporter groups.

Particularly preferred LNA monomer for incorporation into an oligonucleotide of the invention include those of the following formula Ia

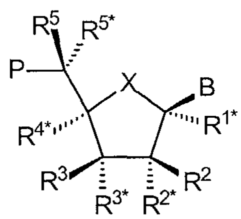

Ia

wherein x is selected among oxygen, sulphur, nitrogen, substituted nitrogen, carbon and substituted carbon, and preferably is oxygen; B is a nucleobase; $R^{1*}$, $R^2$, $R^3$, $R^5$ and $R^{5*}$ are hydrogen; P designates the radical position for an internucleoside linkage to a succeeding monomer, or a 5'-terminal group, $R^{3*}$ is an internucleoside linkage to a preceding monomer, or a 3'-terminal group; and $R^{2*}$ and $R^{4*}$ together designate - O-$CH_2$- where the oxygen is attached in the 2'-position, or a linkage of $-(CH_2)_n-$ where n is 2, 3 or 4, preferably 2, or a linkage of -S-$CH_2$- or -NH-$CH_2$-.

Units of formula Ia where $R^{2*}$ and $R^{4*}$ contain oxygen are sometimes referred to herein as "oxy-LNA"; units of formula Ia where $R^{2*}$ and $R^{4*}$ contain sulphur are sometimes referred to herein as "thio-LNA"; and units of formula Ia where $R^{2*}$ and $R^{4*}$ contain nitrogen are sometimes referred to herein as "amino-LNA". For many applications, oxy-LNA units are preferred modified nucleic acid residues of oligonucleotides of the invention.

As used herein, including with respect to formula Ia, the term "nucleobase" covers the naturally occurring nucleobases adenine (A), guanine (G), cytosine (C), thymine (T) and uracil (U) as well as non-naturally occurring nucleobases such as xanthine, diaminopurine, 8-oxo-$N^6$-methyladenine, 7-deazaxanthine, 7-deazaguanine, $N^4$,$N^4$-ethanocytosin, $N^6$,$N^6$-ethano-2,6-diaminopurine, 5-methylcytosine, 5-($C^3$-$C^6$)-alkynylcytosine, 5-fluorouracil, 5-bromouracil, pseudoisocytosine, 2-hydroxy-5-methyl-4-triazolopyridin, isocytosine, isoguanine, inosine and the "non-naturally occurring" nucleobases described in Benner et al., U.S. Pat No. 5,432,272 and Susan M. Freier and Karl-Heinz Altmann, Nucleic Acids Research, 1997, vol. 25, pp 4429-4443. The term "nucleobase" thus includes not only the known purine and pyrimidine heterocycles, but also heterocyclic analogues and tautomers thereof. It should be clear to the person skilled in the art that various nucleobases, which previously have been considered "non-naturally occurring" have subsequently been found in nature.

A wide variety of modified nucleic acids may be employed, including those that have 2'-modification of hydroxyl, 2'-O-methyl, 2'-fluoro, 2'-trifluoromethyl, 2'-O-(2-methoxyethyl), 2'-O-aminopropyl, 2'-O-dimethylamino-oxyethyl, 2'-O-fluoroethyl or 2'-O-propenyl. The nucleic acid may further include a 3' modification, preferably where the 2'- and 3'-position of the ribose group is linked. The nucleic acid also may contain a modification at the 4'-position, preferably where the 2'-and 4'-positions of the ribose group are linked such as by a 2'-4' link of -$CH_2$-S-, -$CH_2$-NH-, or -$CH_2$-NMe-bridge.

The nucleotide also may have a variety of configurations such as α-D-ribo, β-D-xylo, or α-L-xylo configuration.

The internucleoside linkages of the residues of oligos of the invention may be natural phosphorodi-ester linkages, or other linkages such as -O-P(O)$_2$-O-, -O-P(O,S)-O-, -O-P(S)$_2$-O-, -$NR^H$-P(O)$_2$-O-, -O-P(O,$NR^H$)-O-, -O-PO(R'') -O-, -O-PO($CH_3$)-O-, and -O-PO($NHR^N$)-O-, where $R^H$ is selected form hydrogen and $C_{1-4}$-alkyl, and R'' is selected from $C_{1-6}$-alkyl and phenyl.

In the present context, the term "oligonucleotide" which is the same as "oligomer" which is the same as "oligo"

means a successive chain of nucleoside monomers (*i.e*. glycosides of heterocyclic bases) connected via internucleoside linkages. The linkage between two successive monomers in the oligo consist of 2 to 4, preferably 3, groups/atoms selected from - $CH_2$-, -O-, -S-, -$NR^H$-, >C=O, >C=$NR^H$, >C=S, -Si(R")$_2$-, -SO-, -S(O)$_2$-, -P(O)$_2$-, -PO($BH_3$)-, -P(O,S)-, -P(S)$_2$-, -PO(R")-, -PO($OCH_3$)-, and -PO($NHR^H$)-, where $R^H$ is selected from hydrogen and $C_{1-4}$-alkyl, and R" is selected from $C_{1-6}$-alkyl and phenyl. Illustrative examples of such linkages are -$CH_2$-$CH_2$-$CH_2$-, -$CH_2$-CO-$CH_2$-, - -$CH_2$-CHOH-$CH_2$-, -O-$CH_2$-O-, -O-$CH_2$-$CH_2$-, -O-$CH_2$-CH= (including $R^5$ when used as a linkage to a succeeding monomer) , -$CH_2$-$CH_2$-O-, -$NR^H$-$CH_2$-$CH_2$-, -$CH_2$-$CH_2$-$NR^H$-, -$CH_2$-$NR^H$-$CH_2$-, -O-$CH_2$-$CH_2$-$NR^H$-, -$NR^H$-CO-O-, -$NR^H$-CO-$NR^H$-, -$NR^H$-CS-$NR^H$-, -$NR^H$-C(=$NR^H$)-$NR^H$-, -$NR^H$CO-$CH_2$-$NR^H$-, -O-CO-O-, -O-CO-$CH_2$-O-, -O-$CH_2$-CO-O-, -$CH_2$-CO-$NR^H$-, -O-CO-$NR^H$-, -$NR^H$-CO-$CH_2$-, -O-$CH_2$-CO-$NR^H$-, -O-$CH_2$-$CH_2$-$NR^H$-, -CH=N-O-, -$CH_2$-$NR^H$-O-, -$CH_2$-O-N= (including $R^5$ when used as a linkage to a succeeding monomer), -$CH_2$-O-$NR^H$-, -CO-$NR^H$-$CH_2$-, -$CH_2$-$NR^H$-O-, -$CH_2$-$NR^H$-CO-, -O-$NR^H$-$CH_2$-, -O-$NR^H$-, -O-$CH_2$-S-, -S-$CH_2$-O-, -$CH_2$-$CH_2$-S-, -O-$CH_2$-$CH_2$-S-, -S-$CH_2$-CH= (including $R^5$ when used as a linkage to a succeeding monomer), -S-$CH_2$-$CH_2$-, -S-$CH_2$-$CH_2$-O-, -S-$CH_2$-$CH_2$-S-, -$CH_2$-S-$CH_2$-, -$CH_2$-SO-$CH_2$-, -$CH_2$-$SO_2$-$CH_2$-, -O-SO-O-, -O-S(O)$_2$-O-, -O-S (O)$_2$-$CH_2$-, -O-S(O)$_2$-$NR^H$-, -$NR^H$-S(O)$_2$-$CH_2$-, -O-S(O)$_2$-$CH_2$-, -O-P(O)$_2$-O-, -O-P(O,S)-O-, -O-P(S)$_2$-O-, -S-P(O)$_2$-O-, -S-P(O,S)-O-, -S-P(S)$_2$-O-, -O-P(O)$_2$-S-, -O-P(O,S)-S-, -O-P(S)$_2$-S-, -S-P(O)$_2$-S-, -S-P(O,S)-S-, -S-P(S)$_2$-S-, -O-PO (R")-O-, -O-PO($OCH_3$)-O-, -O-PO($OCH_2CH_3$) -O-, -O-PO($OCH_2CH_2$S-R)-O- , -O-PO($BH_3$)-O-, -O-PO($NHR^N$)-O-, -O-P (O)$_2$-$NR^H$-, -$NR^H$-P(O)$_2$-O-, -O-P(O,$NR^H$)-O-, -$CH_2$-P(O)$_2$-O-, -O-P(O)$_2$-$CH_2$-, and -O-Si(R")$_2$-O-; among which -$CH_2$-CO-$NR^H$-, -$CH_2$-$NR^H$-O-, -S-$CH_2$-O-, -O-P(O)$_2$-O-, -O-P(O,S)-O-, -O-P(S)$_2$-O-, -$NR^H$-P(O)$_2$-O-, -O-P(O,$NR^H$) -O-, -O-PO(R")-O-, -O-PO($CH_3$)-O-, and -O-PO($NHR^N$)-O-, where $R^H$ is selected form hydrogen and $C_{1-4}$-alkyl, and R" is selected from $C_{1-6}$-alkyl and phenyl, are especially preferred. Further illustrative examples are given in Mesmaeker et. al., Current Opinion in Structural Biology 1995, 5, 343-355 and Susan M. Freier and Karl-Heinz Altmann, Nucleic Acids Research, 1997, vol 25, pp 4429-4443. The left-hand side of the internucleoside linkage is bound to the 5-membered ring as substituent $P^*$ at the 3'-position, whereas the right-hand side is bound to the 5'-position of a preceding monomer.

The term "succeeding monomer" relates to the neighboring monomer in the 5'-terminal direction and the "preceding monomer" relates to the neighboring monomer in the 3'-terminal direction.

Monomers are referred to as being "complementary" if they contain nucleobases that can form hydrogen bonds according to Watson-Crick base-pairing rules (e.g. G with C, A with T or A with U) or other hydrogen bonding motifs such as for example diaminopurine with T, inosine with C, pseudoisocytosine with G, etc.

Preferred oligonucleotides of the invention also may have at least one non-modified nucleic acid located either at or within a distance of no more than three bases from the mismatch position(s) of a complementary oligonucleotide, such as at a distance of two bases from the mismatch position, e.g. at a distance of one base from the mismatch position, e.g. at the mismatch position.

The chimeric oligos of the present invention are highly suitable for a variety of diagnostic purposes such as for the isolation, purification, amplification, detection, identification, quantification, or capture of nucleic acids such as DNA, mRNA or non-protein coding cellular RNAs, such as tRNA, rRNA, snRNA and scRNA, or synthetic nucleic acids, *in vivo* or *in vitro.*

The capture oligonucleotide can comprise a photochemically active group, a thermochemically active group, a chelating group, a reporter group, or a ligand that facilitates the direct of indirect detection of the capture oligonucleotide or the immobilization of the capture oligonucleotide onto a solid support. Such group are typically attached to the oligo when it is intended as a probe for *in situ* hybridization, in Southern hybridization, Dot blot hybridization, reverse Dot blot hybridization, or in Northern hybridization.

When the photochemically active group, the thermochemically active group, the chelating group, the reporter group, or the ligand includes a spacer (K), the spacer may suitably comprise a chemically cleavable group.

In the present context, the term "photochemically active groups" covers compounds that are able to undergo chemical reactions upon irradiation with light. Illustrative examples of functional groups hereof are quinones, especially 6-methyl-1,4-naphtoquinone, anthraquinone, naphtoquinone, and 1,4-dimethyl-anthraquinone, diazirines, aromatic azides, benzophenones, psoralens, diazo compounds, and diazirino compounds.

In the present context "thermochemically reactive group" is defined as a functional group, which is able to undergo thermochemically-induced covalent bond formation with other groups. Illustrative examples of functional parts thermochemically reactive groups are carboxylic acids, carboxylic acid esters such as activated esters, carboxylic acid halides such as acid fluorides, acid chlorides, acid bromide, and acid iodides, carboxylic acid azides, carboxylic acid hydrazides, sulfonic acids, sulfonic acid esters, sulfonic acid halides, semicarbazides, thiosemicarbazides, aldehydes, ketones, primary alcohols, secondary alcohols, tertiary alcohols, phenols, alkyl halides, thiols, disulphides, primary amines, secondary amines, tertiary amines, hydrazines, epoxides, maleimides, and boronic acid derivatives.

In the present context, the term "chelating group" means a molecule that contains more than one binding site and frequently binds to another molecule, atom or ion through more than one binding site at the same time. Examples of functional parts of chelating groups are iminodiacetic acid, nitrilotriacetic acid, ethylenediamine tetraacetic acid (EDTA), aminophosphonic acid, etc.

In the present context, the term "reporter group" means a group which is detectable either by itself or as a part of an detection series. Examples of functional parts of reporter groups are biotin, digoxigenin, fluorescent groups (groups which are able to absorb electromagnetic radiation, e.g. light or X-rays, of a certain wavelength, and which subsequently reemits the energy absorbed as radiation of longer wavelength; illustrative examples are dansyl (5-dimethylamino)-1-naphthalenesulfonyl), DOXYL (N-oxyl-4,4-dimethyloxazolidine), Alexa dyes (Molecular Probe Inc.) PROXYL (N-oxyl-2,2,5,5-tetramethylpyrrolidine), TEMPO (N-oxyl-2,2,6,6-tetramethylpiperidine), dinitrophenyl, acridines, coumarins, Cy3 and Cy5 (trademarks for Biological Detection Systems, Inc.), erythrosine, coumaric acid, umbelliferone, Texas red, rhodamine, tetramethyl rhodamine, Rox, 7-nitrobenzo-2-oxa-1-diazole (NBD), pyrene, fluorescein, Europium, Ruthenium, Samarium, and other rare earth metals), radioisotopic labels, chemiluminescence labels (labels that are detectable via the emission of light during a chemical reaction), spin labels (a free radical (*e.g.* substituted organic nitroxides) or other paramagnetic probes (*e.g.* $Cu^{2+}$, $Mg^{2+}$) bound to a biological molecule being detectable by the use of electron spin resonance spectroscopy) , enzymes (such as peroxidases, alkaline phosphatases, β-galactosidases, and glycose oxidases), antigens, antibodies, haptens (groups which are able to combine with an antibody, but which cannot initiate an immune response by itself, such as peptides and steroid hormones), carrier systems for cell membrane penetration such as: fatty acid residues, steroid moieties (cholesteryl), vitamin A, vitamin D, vitamin E, folic acid peptides for specific receptors, groups for mediating endocytose, epidermal growth factor (EGF), bradykinin, and platelet derived growth factor (PDGF). Especially interesting examples are biotin, fluorescein, Texas Red, rhodamine, dinitrophenyl, digoxigenin, Ruthenium, Europium, Cy5, Cy3, etc.

In the present context "ligand" means something which binds. Ligands can comprise functional groups such as: aromatic groups (such as benzene, pyridine, naphthalene, anthracene, and phenanthrene), heteroaromatic groups (such as thiophene, furan, tetrahydrofuran, pyridine, dioxane, and pyrimidine), carboxylic acids, carboxylic acid esters, carboxylic acid halides, carboxylic acid azides, carboxylic acid hydrazides, sulfonic acids, sulfonic acid esters, sulfonic acid halides, semicarbazides, thiosemicarbazides, aldehydes, ketones, primary alcohols, secondary alcohols, tertiary alcohols, phenols, alkyl halides, thiols, disulphides, primary amines, secondary amines, tertiary amines, hydrazines, epoxides, maleimides, $C_1$-$C_{20}$ alkyl groups optionally interrupted or terminated with one or more heteroatoms such as oxygen atoms, nitrogen atoms, and/or sulphur atoms, optionally containing aromatic or mono/polyunsaturated hydrocarbons, polyoxyethylene such as polyethylene glycol, oligo/polyamides such as poly-α-alanine, polyglycine, polylysine, peptides, oligo/polysaccharides, oligo/polyphosphates, toxins, antibiotics, cell poisons, and steroids, and also "affinity ligands", *i.e.* functional groups or biomolecules that have a specific affinity for sites on particular proteins, antibodies, poly- and oligosaccharides, and other biomolecules.

It should be understood that the above-mentioned specific examples under DNA intercalators, photochemically active groups, thermochemically active groups, chelating groups, reporter groups, and ligands correspond to the "active/functional" part of the groups in question. For the person skilled in the art it is furthermore clear that DNA intercalators, photochemically active groups, thermochemically active groups, chelating groups, reporter groups, and ligands are typically represented in the form M-K- where M is the "active/functional" part of the group in question and where K is a spacer through which the "active/functional" part is attached to the 5- or 6-membered ring. Thus, it should be understood that the group B, in the case where B is selected from DNA intercalators, photochemically active groups, thermochemically active groups, chelating groups, reporter groups, and ligands, has the form M-K-, where M is the "active/functional" part of the DNA intercalator, photochemically active group, thermochemically active group, chelating group, reporter group, and ligand, respectively, and where K is an optional spacer comprising 1-50 atoms, preferably 1-30 atoms, in particular 1-15 atoms, between the 5- or 6-membered ring and the "active/functional" part.

In the present context, the term "spacer" means a thermochemically and photochemically non-active distance-making group and is used to join two or more different moieties of the types defined above. Spacers are selected on the basis of a variety of characteristics including their hydrophobicity , hydrophilicity, molecular flexibility and length (*e. g.* see Hermanson et. al., "Immobilized Affinity Ligand Techniques", Academic Press, San Diego, California (1992), p. 137-ff). Generally, the lengths of the spacers are less than or about 400 Å, in some applications preferably less than 100 Å. The spacer, thus, comprises a chain of carbon atoms optionally interrupted or terminated with one or more heteroatoms, such as oxygen atoms, nitrogen atoms, and/or sulphur atoms. Thus, the spacer K may comprise one or more amide, ester, amino, ether, and/or thioether functionalities, and optionally aromatic or mono/polyunsaturated hydrocarbons, polyoxyethylene such as polyethylene glycol, oligo/polyamides such as poly-α-alanine, polyglycine, polylysine, and peptides in general, oligosaccharides, oligo/polyphosphates. Moreover the spacer may consist of combined units thereof. The length of the spacer may vary, taking into consideration the desired or necessary positioning and spatial orientation of the "active/functional" part of the group in question in relation to the 5- or 6-membered ring. In particularly interesting embodiments, the spacer includes a chemically cleavable group. Examples of such chemically cleavable groups include disulphide groups cleavable under reductive conditions, peptide fragments cleavable by peptidases, etc.

Kits are also provided containing one or more capture oligonucleotides of the invention for the detection of

one or more particular sequences and one or more helper probes suited for enhancing the binding of said one or more capture oligonucleotides. In case that the capture oligonucleotide is labelled the kit may also comprise reagents suitable for detection of said label. The kit typically will contain a reaction body, e.g. a slide or biochip. One or more oligonucleotides of the invention may be suitably immobilized on such a substrate platform.

The invention also provides methods for using kits of the invention for carrying out a variety of bioassays. Any type of assay wherein one component is immobilized may be carried out using the substrate platforms of the invention. Bioassays utilizing an immobilized component are well known in the art. Examples of assays utilizing an immobilized component include for example, immunoassays, analysis of protein-protein interactions, analysis of protein-nucleic acid interactions, analysis of nucleic acid-nucleic acid interactions, receptor binding assays, enzyme assays, phosphorylation assays, diagnostic assays for determination of disease state, genetic profiling for drug compatibility analysis, SNP detection, etc.

Identification of a nucleic acid sequence capable of binding to a biomolecule of interest can be achieved by immobilizing a library of nucleic acids onto the substrate surface so that each unique nucleic acid was located at a defined position to form an array. The array would then be exposed to the biomolecule under conditions which favored binding of the biomolecule to the nucleic acids. Non-specifically binding biomolecules could be washed away using mild to stringent buffer conditions depending on the level of specificity of binding desired. The nucleic acid array would then be analyzed to determine which nucleic acid sequences bound to the biomolecule. Preferably the biomolecules would carry a fluorescent tag for use in detection of the location of the bound nucleic acids.

Assay using an immobilized array of nucleic acid sequences may be used for determining the sequence of an unknown nucleic acid; single nucleotide polymorphism (SNP) analysis; analysis of gene expression patterns from a particular species, tissue, cell type, etc.; gene identification; etc.

The capture probes or oligonucleotides used in the methods of the present invention may be used without any prior analysis of the structure assumed by a target nucleic acid. For any given case, it can be determined empirically using appropriately selected reference target molecule whether a chosen probe or array of probes can distinguish between genetic variants sufficiently for the needs of a particular assay. Once a probe or array of probes is selected, the analysis of which probes bind to a target, and how efficiently these probes bind (i.e., how much of probe/target complex can be detected) allows a hybridization signature of the conformation of the target to be created. It is contemplated that the signature may be stored, represented or analyzed by any of the methods commonly used for the presentation of mathematical and physical information, including but not limited to line, pie, or area graphs or 3-dimensional topographic representations. The data may also be used as a numerical matrix, or any other format that may be analyzed either visually, mathematically or by computer-assisted algorithms, such as for example EURAYdesign™ software and/or neural networks.

The resulting signatures of the nucleic acid structures serve as sequence-specific identifiers of the particular molecule, without requiring the determination of the actual nucleotide sequence. While specific sequences may be identified by comparison of their signature to a reference signature, the use of algorithms to deduce the actual sequence of a molecule by sequence-specific hybridization (i.e., at high stringency to eliminate the influence of secondary and tertiary structures) to a complete matrix (i.e., probes that shift by a single nucleotide position at each location of an array), is not a feature or requirement, or within the bounds of the methods of the present invention.

It is also contemplated that information on the structures assumed by a target nucleic acid may be used in the design of the probes, such that regions that are known or suspected to be involved in folding may be chosen as hybridization sites. Such an approach will reduce the number of probes that are likely to be needed to distinguish between targets of interest.

There are many methods used to obtain structural information involving nucleic acids, including the use of chemicals that are sensitive to the nucleic acid structure, such as phenanthroline/copper, EDTA-$Fe^{2+}$, cisplatin, ethylnitrosourea, dimethyl pyrocarbonate, hydrazine, dimethyl sulfate, and bisulfite. Enzymatic probing using structure-specific nucleases from a variety of sources, such as the Cleavase™ en- zymes (Third Wave Technologies, Inc., Madison, Wis.), Taq DNA polymerase, E. coli DNA polymerase I, and eukaryotic structure-specific endonucleases (e.g., human, murine and Xenopus XPG enzymes, yeast RAD2 enzymes), murine FEN-1 endonucleases (Harrington and Lieber, Genes and Develop., 3:1344 [1994]) and calf thymus 5' to 3' exonuclease (Murante et al., J. Biol. Chem., 269: 1191 [1994]). In addition, enzymes having 3' nuclease activity such as members of the family of DNA repair endonucleases (e.g., the RrpI enzyme from Drosophila melanogaster, the yeast RAD1/RAD10 complex and E. coli Exo III), are also suitable for examining the structures of nucleic acids.

If analysis of structure as a step in probe selection is to be used for a segment of nucleic acid for which no information is available concerning regions likely to form secondary structures, the sites of structure-induced modification or cleavage must be identified. It is most convenient if the modification or cleavage can be done under partially reactive conditions (i.e., such that in the population of molecules in a test sample, each individual will receive only one or a few cuts or modifications) . When the sample is analyzed as a whole, each reactive site should be represented, and all the sites may be thus identified. Using a Cleavase Fragment Length Polymorphism™ cleavage reaction as an

example, when the partial cleavage products of an end labeled nucleic acid fragment are resolved by size (e.g., by electrophoresis), the result is a ladder of bands indicating the site of each cleavage, measured from the labeled end. Similar analysis can be done for chemical modifications that block DNA synthesis; extension of a primer on molecules that have been partially modified will yield a nested set of termination products. Determining the sites of cleavage/ modification may be done with some degree of accuracy by comparing the products to size markers (e.g., commercially available fragments of DNA for size comparison) but a more accurate measure is to create a DNA sequencing ladder for the same segment of nucleic acid to resolve alongside the test sample. This allows rapid identification of the precise site of cleavage or modification.

The capture oligonucleotides may interact with the target in any number of ways. For example, in another embodiment, the capture oligonucleotides may contact more than one region of the target nucleic acid. When the target nucleic acid is folded as described, two or more of the regions that remain single stranded may be sufficiently proximal to allow contact with a single capture probe. The capture oligonucleotide in such a configuration is referred to herein as a "bridge" or "bridging" oligonucleotide, to reflect the fact that it may interact with distal regions within the target nucleic acid. The use of the terms "bridge" and "bridging" is not intended to limit these distal interactions to any particular type of interaction. It is contemplated that these interactions may include non-standard nucleic acid interactions known in the art, such as G-T base pairs, Hoogsteen interactions, triplex structures, quadraplex aggregates, and the multibase hydrogen bonding such as is observed within nucleic acid tertiary structures, such as those found in tRNAs. The terms are also not intended to indicate any particular spatial orientation of the regions of interaction on the target strand, i. e., it is not intended that the order of the contact regions in a bridge oligonucleotide be required to be in the same sequential order as the corresponding contact regions in the target strand. The order may be inverted or otherwise shuffled.

The invention is further illustrated by the following examples, which are provided solely for illustration of the invention and should not be regarded as limiting in any way.

## EXAMPLES

### Example 1

### The use of LNA helper probes to improve the capture of single-stranded DNA targets by immobilized anthraquinone-coupled LNA capture probes

The present method describes the use of LNA oligonucleotides as helper probes to improve the capture of single-stranded DNA targets by immobilized LNA capture probes.

LNA-modified oligonucleotides (see table I) carrying a 5'-anthraquinone were covalently immobilized to the wells of a microtiter-plate by UV irradiation and used as capture probes in a hybridization assay with a complementary DNA target oligonucleotide. The hybrid was detected by including an LNA helper probe in the hybridization mixture.

Table I.

| Oligonucleotides used in the example. | | | |
|---|---|---|---|
| Name | Short notation | Sequence | Characteristics |
| ApoE112C-8 | 112C | 5'-AQ2-nbnbnb nbnbnb nbnbnb nbnbnb nbnbnb $C^{met}$ $GC^{met}G$-$C^{met}AC^{met}Gt$-3' | Capture probe |
| ApoE112T-8 | 112T | 5'-AQ2-nbnbnb nbnbnb nbnbnb nbnbnb nbnbnb $C^{met}$ $GC^{met}AC$-$^{met}AC^{met}Gt$-3' | Capture probe |
| hj-LNA 11'mer as 5' end | LNA 1 | 5'-$C^{met}TC^{met}C^{met}ATGTC^{met}C^{met}G$-3' | LNA helper probe |
| hj-LNA 11'mer as 3' end | LNA 2 | 5'-$AC^{met}TGC^{met}AC^{met}C^{met}AGG$-3' | LNA helper probe |
| hj-LNA 11'mer as 5' end | LNA 3 | 5'-$AC^{met}ATGGAGGAC^{met}$-3' | LNA helper probe |
| hj-LNA 11'mer as 3' end | LNA 4 | 5'-$GC^{met}$ $C^{met}GC^{met}C^{met}TGGTG$-3' | LNA helper probe |

Table I. (continued)

| Oligonucleotides used in the example. | | | |
|---|---|---|---|
| Name | Short notation | Sequence | Characteristics |
| hj-DNA 11'mer as 5' end | DNA 1 | 5'-ctccatgtccg-3' | DNA helper probe |
| hj-DNA 11'mer as 3' end | DNA 2 | 5'-actgcaccagg-3' | DNA helper probe |
| hj-DNA 11'mer as 5' end | DNA 3 | 5'-acatggaggac-3' | DNA helper probe |
| hj-DNA 11'mer as 3' end | DNA 4 | 5'-gccgcctggtg-3' | DNA helper probe |
| 50mer targetoligo 112c | EQ 3309 | 5'-biotin-ggcgcggacatggaggacgtgc gcggccgcctggtgcagtaccgcggcga-3' | 5' biotinylated DNA target |
| 50mer targetoligo 112t | EQ 3313 | 5'-biotin-ggcgcggacatggaggacgtgt gcggccgcctggtgcagtaccgcggcga-3' | 5' biotinylated DNA target |
| Note: LNA nucleotides are indicated with uppercase letters, DNA nucleotides are indicated by lowercase letters. $C^{met}$ indicates 5-methyl cytosine LNA; nb indicates a DNA non-base; 5'-AQ2 indicates that the oligonucleotide carries a 5'anthraquinone (AQ) and a C3 linker, the composition is AQ-CONH-$(CH_2)_3$-oligonucleotide. 5'-biotin indicates 5' biotin-$(CH_2)_4$-CONH-$(CH_2)_6$-oligo. | | | |

Immobilized Capture Probes:

Anthraquinone-coupled LNA capture probes (either ApoE112C-8 or ApoE112T-8, see table I) were dissolved in pure Milli-Q water and the concentration was determined by UV absorbance at 260 nm using the extension coefficient for DNA. From these stock solutions the final concentration at were made. The capture probes were diluted in 0.2 M NaCl at a concentration of 0.3 μM. 100 μL aliquots of the oligonucleotides were added to the wells of the microtiter-plate (C96 polysorp; Nalge Nunc International, Roskilde, Denmark), and exposed for 15 min. to soft UV light (approximately 350 nm) in a ULS-20-2 illuminator (UV-Lights Systems, Denmark) at 35°C. The illuminator was equipped with 28 Philips Cleo Compact 25W-S light bulbs (14 located above and 14 located below the glass plate sample holder) only the upper bulbs were lit.

After incubation, the wells were incubated with 300 μL 0.4 M NaOH containing 0.25 % Tween 20 (Riedel-de Häen, Seelze, Germany) for 5 minutes at room temperature and then washed three times with 300 μL deionized water.

Hybridization with target molecules differing by a single nucleotide

To wells coated with either the ApoE112C-8 capture probe (112C) or ApoE112T-8 capture probe (112T), 50mer target oligonucleotide 112c (EQ 3309) or 112t (EQ 3313) were added in a constant concentration (1 μM) while the amount of helper probe was varied in a three-fold dilution series. Four different LNA helper probes (EQ 3828, EQ3829, EQ3830, and EQ3831) and four DNA helper probes (EQ 3842, EQ3843, EQ3844, and EQ3845) were tested. The resulting concentration of each helper probe was 0.0041, 0.012, 0.037, 0.111, 0.333, 1, 3 and 9 μM, respectively.

The target oligonucleotide and the helper oligonucleotide were mixed in a volume of 20 μL. 20 μL of denaturation buffer (80 mM EDTA pH 8.0, 125 mM NaOH, phenol red) was added and the mixture was incubated for 5 minutes at room temperature. 200 μL of hybridization buffer was added and the mixture was vortexed. 100 μL aliquots of the hybridization mix were added to microtiter-plate wells containing either ApoE112C-8 or ApoE112T-8. The capture, target and helper oligos were allowed to hybridizes for one hour at 37°C. The wells were washed five times with 300 μL 0.5 x SSC containing 0.1 % Tween 20 (0.5 x SSC is: 75 mM NaCl, 7.5 mM sodium citrate).

The hybrids were detected by binding streptavidin-horse radish peroxidase to the biotinylated detection probe. The strA-HRP (Pierce, Rockford, IL, USA. Cat. no. 21126) was dissolved in 0.5 x SSC containing 0.1 % Tween 20 at a concentration of 1 μg/mL. 100 μL aliquots were added per well and the microplate was incubated for 15 min. The plate was then washed five times with 0.5 x SSC; 0.1 % Tween 20 and the hybridization signals were detected using the OPD-assay.

OPD-assay:

A master-mix containing: 12 mL 0.1 M citrate buffer pH=5.0, four 2 mg ortho-phenylene-diamine (OPD) tablets (Kem-En-Tec, Copenhagen, Denmark, cat no. 4090) and 5 µL 30 % $H_2O_2$ was prepared. 100 µL of the master-mix was added to each reaction well and the plate was incubated for one to 30 min. depending on the enzyme activity. The assay was stopped with 100 µL of 0.5 M $H_2SO_4$ and the optical density was measured at 492 nm with an ELISA-reader.
The results are shown in figures 1A, 1B, 1C, and 1D.
In figure 1A it is shown that one of the helper probes (LNA 1) increases the ability of the immobilized capture probe 112 C to bind to the target sequence EQ 3309 in a concentration dependent manner. The LNA 1 helper probe also shows an ability to increase the capture of the target EQ 3313, however, less pronounced.
The LNA 2 helper probe show a tendency to decrease the binding of the target EQ 3313 to the immobilized capture probe 112 C. Thus, it is possible to modulate the hybridisation characteristics between capture and target by the design of the helper probe.
In figure 1B, the LNA 1 helper probe shows, in a high concentration, a destabilizing effect on the capture of the target EQ 3313. For Figs. 1C and 1D, no general trend can be derived.

**Example 2**

**The use of LNA helper probes for the specific capture of PCR amplicons by immobilized anthraquinone-coupled LNA capture probes.**

To improve the signal for the specific capture of PCR amplicons, a LNA oligonucleotide was used as a helper probe.
LNA modified oligonucleotides (see Table II) carrying a 5'anthraquinone were covalently immobilized to the wells of a microtiter-plate by UV irradiation and used as capture probes in a hybridization assay with a complementary double-stranded DNA target. The hybrid was detected by including an LNA or DNA helper probe in the hybridization mixture.

Table II:

| Oligonucleotides used in the example. | | | |
|---|---|---|---|
| Name | Short notation | Sequence | Characteristics |
| ApoE112C-8 | 112C | 5'-AQ2-nbnbnb nbnbnb nbnbnb nbnbnb nbnbnb $C^{met}$ $GC^{met}GC^{met}AC^{met}Gt$-3' | Capture probe |
| ApoE112T-8 | 112T | 5'-AQ2-nbnbnb nbnbnb nbnbnb nbnbnb nbnbnb $C^{met}$ $GC^{met}AC^{met}AC^{met}Gt$-3' | Capture probe |
| Bio-ApoE-s-112 | EQ 3730 | 5'-biotin-ggcgcggacatggaggac-3' | 5' biotinylated forward primer |
| ApoE-as-112 | EQ 3886 | 5'-tgcacctcgccgcggtac-3' | reverse primer |
| hj-LNA 11'mer as 5' end | LNA 1 | 5'-$C^{met}TC^{met}C^{met}ATGTC^{met}C^{met}G$-3' | LNA helper probe |
| hj-DNA 11'mer as 5' end | DNA 1 | 5'-ctccatgtccg-3' | DNA helper probe |
| 50'mer target oligo 112c | Target 112c | 5'-biotin-ggcgcggacatggaggacgtgc gcggccgcctggtgcagtaccgcggcga-3' | 5' biotinylated DNA target |
| 50'mer target oligo 112t | Target 112t | 5'-biotin-ggcgcggacatggaggacgtgt gcggccgcctggtgcagtaccgcggcga-3' | 5' biotinylated DNA target |
| Note: LNA nucleotides are indicated with uppercase letters, DNA nucleotides by lowercase letters. $C^{met}$ is 5-methyl cytosine LNA, nb indicates a DNA non-base. 5'-AQ2 indicates that the oligonucleotide carries a 5'anthraquinone (AQ) and a C3 linker, the composition is AQ-CONH-$(CH_2)_3$-oligo. 5'-biotin indicates 5' biotin-$(CH_2)_4$-CONH-$(CH_2)_6$-oligo. | | | |

Immobilized Capture Probes:

Anthraquinone-coupled LNA capture probes (either ApoE112C-8 or ApoE112T-8, see table II) were dissolved in pure Milli-Q water and the concentrations were determined by UV absorbance at 260 nm using the extensions coefficients for DNA. The capture probes were diluted in 0.2 M NaCl at a final concentration of 0.3 µM. 100 µL aliquots of the oligonucleotides were added the wells of the microtiter-plate (C96 polysorp; Nalge Nunc International, Roskilde, Denmark) , and the plate was exposed for 15 min. to soft UV light (approximately 350 nm) in a ULS-20-2 illuminator (UV-Lights Systems, Denmark) at maximal 35°C. The illuminator was equipped with 28 Philips Cleo Compact 25W-S light bulbs (14 located above and 14 located below the glass plate sample holder) only the upper bulbs were lit. After incubation the wells were incubated with first 300 µL 0.4 M NaOH containing 0.25 % Tween 20 (Riedel-de Häen, Seelze, Germany) for 5 minutes at room temperature and then washed three times with 300 µL deionized water.

PCR amplification

PCR master-mix for 20 reactions @ 50 µL:

697 µL $H_2O$
100 µL 10xAmpliTaq Gold buffer (Perkin-Elmer Corporation, Norwalk, CT, USA).
80 µL $MgCl_2$ (25 mM)
50 µL dNTP (2 mM)
30 µL forward primer EQ3730 (10 µM)
30 µL reverse primer EQ3886 (10 µM)
5 µL AmpliTaq Gold® DNA Polymerase (5 U/µL) (Perkin Elmer cat. no. N808-0240, Perkin-Elmer Corporation, Norwalk, CT, USA).

The PCR reactions were carried out in 0.2 mL thin-wall tubes using an Eppendorf Mastercycler Gradient thermocycler (Eppendorf - Netheler - Hinz GmbH, Hamburg, Germany) . To 49.5 µL aliquots of the PCR master-mix 0.5 µL template was added.

Synthesis and analysis of primers:

DNA primers were obtained as HPLC purified oligonucleotides from a commercial source (DNA Technology, Aarhus, Denmark) .

Templates:

0.5 nM of either target 112c or target 112t were used as templates.

PCR reaction conditions:

| | |
|---|---|
| Denaturation | 94°C, 15 minutes |
| Amplification (35 cycles) | 94°C, 30 seconds; 65°C, 30 seconds |
| Elongation | 72°C, 10 minutes |
| Termination | 4°C, . |

Detection:

Using the forward and reverse primers applied on the templates, described above, the expected PCR amplicon is 58-basepairs with a 5' biotin on the sense strand. The PCR products were analyzed by standard gel electrophoresis on a 2% agarose gel (LE, Analytical Grade; Promega Corporation, Madison, USA) including GelStar® (FMC BioProducts, Rockland, ME USA) diluted 1:30.000 in the gel using 1 x Tris-acetate/EDTA electrophoresis buffer (0.04 M Tris-acetate; 0.001 M EDTA). To 5 µL of each PCR reactions 1 µL of 6 x loading buffer (40% sucrose, 0.25% bromophenol blue, 0.25% xylene cyanol, 0.1 M EDTA pH 8.0) were added). The gel was run for approximately one hour at a constant voltage of 7 V/cm.

The PCR products were visualized using a standard Polaroid (Polaroid LTD., St. Albans, UK) photography

using an appropriate UV-transilluminator (Model TM-20E UV Products, Upland, CA, USA) and filter (Kodak Wratten #9 Eastman Kodak Co., Rochester, NY, USA).

Hybridization with double stranded target molecules differing by a single nucleotide

To the microplate wells coated with either the ApoE112C-8 capture probe (EQ 3501) or the ApoE112T-8 capture probe (EQ 3625), 10 µl of the PCR product from above were added, while the concentration of the enhancer probe was varied in a three-fold dilution series. One LNA helper probe (LNA1) and one DNA control probe (DNA1) were tested. The resulting concentrations of each helper probe were 2E-4, 5E-4, 0.001, 0.0041, 0.012, 0.037, 0.111, 0.333, 1, 3 and 9 µM.

Target PCR amplicons and the helper oligo were mixed in a total volume of 20 µL. 20 µL of denaturation buffer (80 mM EDTA pH 8.0, 125 mM NaOH, phenol red) was added to the mixture followed by incubation for 5 minutes at room temperature. 200 µL of hybridization buffer was added and the mixture was vortexed. 100 µL aliquots of the hybridization mix were added to a microtiter-plate well containing either ApoE112C-8 or ApoE112T-8. The capture, target and helper oligonucleotides were allowed to hybridize for an hour at 37°C. The wells were washed five times with 300 µL 0.5 x SSC containing 0.1 % Tween 20 (0.5 x SSC is: 75 mM NaCl, 7.5 mM sodium citrate).

The hybrids were detected by binding streptavidin-horse radish peroxidase to the biotinylated detection probe. The strA-HRP (Pierce, Rockford, IL, USA. Cat. no. 21126) was dissolved in 0.5 x SSC with 0.1 % Tween 20 at a concentration of 1 µg/mL. 100 µL was added per well and the plate was incubated for 15 min. The plate was subsequently washed five times with 0.5 x SSC; 0.1 % Tween 20 and the hybridization signals were developed using the OPD-assay described below.

OPD-assay:

A master-mix containing: 12 mL of 0.1 M citrate buffer pH=5.0, four 2 mg ortho-phenylene-diamine (OPD) tablets (Kem-En-Tec, Copenhagen, Denmark, cat no. 4090) and 5 µL 30 % $H_2O_2$ was prepared. 100 µL aliquots of the master-mix was added to each reaction well and the plate was left to incubate for one to 30 min. depending on the enzyme activity. The assay was stopped with 100 µL of 0.5 M $H_2SO_4$ and the optical density was measured at 492 nm using an ELISA-reader.

The results are shown in Figure 2 indicate that the LNA helper probe enhances the capture of the matching target compared to the corresponding DNA helper probe at a lower concentration. Furthermore, it is shown that it is possible at a relative low concentration of the LNA helper probe to capture a double stranded matching target nucleotide. Still further, the results indicate that the capturing immobilized probe discriminates between a matching target and a target having a single mismatching nucleotide.

**Example 3**

**The use of LNA helper probes for the specific capture of PCR amplicons based on patient samples.**

To improve the signal for the specific capture of PCR amplicons, a LNA oligonucleotide was used as a helper probe.

LNA-modified oligonucleotides (see Table III) carrying a 5'anthraquinone were covalently immobilized to the wells of a microtiter-plate by UV irradiation and used as capture probes in a hybridization assay with a complementary double-stranded DNA target. The hybrid was detected by including a LNA helper probe in the hybridization mixture.

Table III:

| Oligonucleotides used in the example. | | | |
|---|---|---|---|
| Name | Short notation | Sequence | Characteristics |
| ApoE112C-8 | 112C | 5'-AQ2-nbnbnb nbnbnb nbnbnb nbnbnbnbnbnb $C^{met}$ $GC^{met}GC^{met}AC^{met}Gt$-3' | 5' anthraquinone modified LNA |
| ApoE112T-8 | 112T | 5'-AQ2-nbnbnb nbnbnb nbnbnb nbnbnb nbnbnb $C^{met}$ $GC^{met}AC^{met}AC^{met}Gt$-3' | 5' anthraquinone modified LNA |

Table III: (continued)

| Oligonucleotides used in the example. | | | |
|---|---|---|---|
| Name | Short notation | Sequence | Characteristics |
| Bio-ApoE-s-112 | EQ 3730 | 5'-biotin-ggcgcggacatggaggac-3' | 5' biotinylated forward primer |
| ApoE-as-112 | EQ 3886 | 5'-tgcacctcgccgcggtac-3' | reverse primer |
| hj-LNA 11'mer as 5' end | LNA 1 | 5'-$C^{met}$ $TC^{met}C^{met}ATGTC^{met}$ $C^{met}G$-3' | LNA helper probe |
| hj-LNA 10'mer as 5' end | LNA 5 | 5'-$C^{met}TC^{met}C^{met}ATGTC^{met}C^{met}$-3' | LNA helper probe |
| hj-LNA gapmer as 5' end | LNA 6 | 5'-$C^{met}TC^{met}C^{met}AtgTC^{met}C^{met}G$-3' | LNA helper probe |
| Note: LNA nucleotides are indicated with uppercase letters, DNA nucleotides by lowercase letters. $C^{met}$ is 5-methyl cytosine LNA, nb indicates a DNA non-base. 5'-AQ2 indicates that the oligo carries a 5'anthraquinone (AQ) and a C3 linker, the composition is AQ-CONH-$(CH_2)_3$-oligonucleotide. 5'-biotin indicates 5' biotin-$(CH_2)_4$-CONH-$(CH_2)_6$-oligo. | | | |

Immobilized Capture Probes:

Anthraquinone-coupled LNA capture probes (either ApoE112C-8 or ApoE112T-8, see table III) were dissolved in pure Milli-Q water and the concentrations were determined by UV absorbance at 260 nm using the extension coefficients for DNA. The capture probes were diluted in 0.2 M NaCl at a final concentration of 0.3 µM. 100 µL aliquots of the oligonucleotides were added the wells of the microtiter-plate (C96 polysorp; Nalge Nunc International, Roskilde, Denmark), and the plate was exposed for 15 min. to soft UV light (approximately 350 nm) in a ULS-20-2 illuminator (UV-Lights Systems, Denmark) at maximal 35°C. The illuminator was equipped with 28 Philips Cleo Compact 25W-S light bulbs (14 located above and 14 located below the glass plate sample holder) only the upper bulbs were lit. After incubation the wells were incubated first with 300 µL 0.4 M NaOH containing 0.25 % Tween 20 (Riedel-de Häen, Seelze, Germany) for 5 minutes at room temperature and then washed three times with 300 µL deionized water.

PCR amplification

PCR master-mix for 6 reactions @ 50 µL:

199.5 µL $H_2O$
30 µL 10xAmpliTaq Gold buffer (Applied Biosystem cat. no. 4311814, Roche Molecular Systems Inc.)
24 µL $MgCl_2$ (25 mM)
15 µL dNTP (2 mM)
9 µL forward primer EQ3730 (10 µM)
9 µL reverse primer EQ3886 (10 µM)
1.5 µL AmpliTaq Gold® DNA Polymerase (5 U/µL) (Applied Biosystem cat. no. 4311814, Roche Molecular Systems Inc.)

The PCR reactions were carried out in 0.2 mL thin-wall tubes using an Eppendorf Mastercycler Gradient thermocycler (Eppendorf - Netheler - Hinz GmbH, Hamburg, Germany). To 48 µL aliquots of the PCR master-mix 2 µL template was added.

Synthesis and analysis of primers:

DNA primers were obtained as HPLC-purified oligonucleotides from a commercial source (DNA Technology, Aarhus, Denmark).

Templates:

200 ng genomic DNA was purified from EDTA blood.
KBA no. #TV130, #TV139, #TV140, #TV142, and blank control.

PCR reaction conditions:

| | |
|---|---|
| Denaturation | 94°C, 15 minutes |
| Amplification (35 cycles) | 94°C, 30 seconds; 65°C, 30 seconds7 |
| Elongation | 72°C, 10 minutes |
| Termination | 4°C, |

Detection:

Using the forward and reverse primers applied on the templates, described above, the expected PCR amplicon is 58-basepairs with a 5' biotin on the sense strand. The PCR products were analyzed by standard gel electrophoresis on a 2% agarose gel (LE, Analytical Grade; Promega Corporation, Madison, USA) including GelStar® (FMC BioProducts, Rockland, ME USA) diluted 1:30.000 in the gel using 1 x Tris-acetate/EDTA electrophoresis buffer (0.04 M Tris-acetate; 0.001 M EDTA). To 5 µL of each PCR reactions 1 µL of 6 x loading buffer (40% sucrose, 0.25% bromophenol blue, 0.25% xylene cyanol, 0.1 M EDTA pH 8.0) were added). The gel was run for approximately one hour at a constant voltage of 7 V/cm.

The PCR products were visualized using a standard Polaroid (Polaroid LTD., St. Albans, UK) photography using an appropriate UV-transilluminator (Model TM-20E UV Products, Upland, CA, USA) and filter (Kodak Wratten #9 Eastman Kodak Co., Rochester, NY, USA).

Hybridization with double stranded target molecules differing by a single nucleotide

To the microplate wells coated with either the ApoE112C-8 capture probe (EQ 3501) or the ApoE112T-8 capture probe (EQ 3625), 10 µl of the PCR product from above were added, while the concentration of the enhancer probe was varied in a three-fold dilution series. Three different LNA helper probes (EQ 3828, EQ4175, and EQ4190) were tested in a final concentration of 0.020 µM.

Target PCR amplicons and the helper oligo were mixed in a total volume of 20 µL. 20 µL of denaturation buffer (80 mM EDTA pH 8.0, 125 mM NaOH, phenol red) was added to the mixture followed by incubation for 5 minutes at room temperature. 200 µL of hybridization buffer was added and the mixture was vortexed. 100 µL aliquots of the hybridization mix were added to a microtiter-plate well containing either ApoE112C-8 or ApoE112T-8. The capture, target and enhancer oligonucleotides were allowed to hybridize for an hour at 37°C. The wells were washed five times with 300 µL 0.5 x SSC containing 0.1 % Tween 20 (0.5 x SSC is: 75 mM NaCl, 7.5 mM sodium citrate).

The hybrids were detected by binding streptavidin-horse radish peroxidase to the biotinylated detection probe. The strA-HRP (Pierce, Rockford, IL, USA. Cat. no. 21126) was dissolved in 0.5 x SSC with 0.1 % Tween 20 at a concentration of 1 µg/mL. 100 µL was added per well and the plate was incubated for 15 min. The plate was subsequently washed five times with 0.5 x SSC; 0.1 % Tween 20 and the hybridization signals were developed using the TMB-assay described below.

TMB-assay:

100 µL 3,3',5,5'-tetramethylbenzidine (TMB one "ready to use") substrate (Kem-En-Tec, Copenhagen, Denmark, cat no. 4380) was added to each reaction well and subsequently incubated for 10 - 15 minutes depending of enzyme activity. The assay was stopped with 100 µL of 0.5 M $H_2SO_4$ and the optical density was measured at 450 nm with an ELISA-reader (Wallac-Victor).

The results are shown in Figure 3. The results indicate that it is possible to determine the genotype of a subject. For patients #TV130 and #TV142 the individuals are homozygotic due to the preferred hybridization to the immobilized capture probe 112C. The results for patients #TV139 and #TV140 indicate for the LNA 1 and LNA 5 helper probes, that the patients, within acceptable tolerances, may be determined as heterocygic, whereas the results for the helper probe LNA 6 indicate that the patients may be homozygotic. Further investigations should be performed in order to determine the genotype of patients #TV139 and #TV140. The results also indicate that eh shortage of the helper probe from 11 nucleotides to 10 nucleotides increases the sensitivity of the assay.

The invention has been described in detail with reference to preferred embodiments thereof. However, it will be appreciated that those skilled in the art, upon consideration of this disclosure, may make modifications and improvements within the spirit and scope of the invention.

## Claims

1. A method for detection of a nucleotide target sequence in a sample by hybridisation using a hybridisation mixture comprising a capture oligonucleotide and a helper probe capable of enhancing the binding of said capture oligonucleotide to said nucleotide target sequence, wherein the helper probe is an oligonucleotide comprising modified nucleic acid residues.

2. The method according to claim 1, wherein the capture oligonucleotide comprises modified nucleic acid residues.

3. The method according to any of the claims 1 or 2, wherein the capture oligonucleotide is immobilized.

4. The method according to any of the claims 1 to 3, wherein the helper probe is capable of reshaping the secondary structure of the nucleotide target around the nucleotide target sequence.

5. The method according to any of the claims 1 to 4, wherein the helper probe is capable of invading a double stranded molecule in the region of the target sequence by denaturising the bonds between the target sequence and the complementary sequence thereof.

6. The method according any of the claims 1 to 5, wherein the helper probe has a higher specificity and affinity for a target nucleotide sequence than a complementary DNA target nucleotide sequence.

7. The method of any of the preceding claims, wherein the helper probe comprises a mixture of modified and non-modified nucleic acid residues.

8. The method according to claim 7, wherein greater than 50 percent of the total residues of the helper probe are modified nucleic acids.

9. The method according to any of the claims 1-8, wherein the helper probe does not contain more than six consecutive modified nucleic acid residues.

10. The method according to any of the claims 1-9, wherein the helper probe is a gabmer.

11. The method according to any of the claims 1-10, wherein the helper probe contains from 4 to 100 total residues.

12. The method according to claim 11, wherein the helper probe contains from 4 to 50 total residues.

13. The method according to claim 12, wherein the helper probe contains from 4 to 30 total residues.

14. The method according to claim 13, wherein the helper probe contains from 8 to 15 total residues.

15. The method according to any of the claims 1 to 14, wherein a modified nucleic acid residue of the helper probe or the capture oligonucleotide contains a modification at the 2'-position in the ribose.

16. The method according to any of the claims 1 to 15 wherein one or more of the modified nucleic acid residues of the helper probe or capture oligonucleotide is LNA-residues.

17. The method of claim 16, wherein the LNA residue is an oxy-LNA residue.

18. The method according to claim 15, wherein one or more modified residues independently are selected among the group consisting of 2'-deoxy-2'-fluoro ribonucleotides, 2'-*O*-methyl ribonucleotides, 2'-*O*-methoxyethyl ribonucleotides, peptide nucleic acids, 5-propynyl pyrimidine ribonucleotides, 7-deazapurine ribonucleotides, 2,6-diaminopurine ribonucleotides, and 2-thio-pyrimidine ribonucleotides.

19. The method according to any of the preceding claims, wherein the non-modified residues contain deoxyribonucleotides.

20. The method according to any of the preceding claims, wherein the capture oligonucleotide is conjugated to a reporter group.

**21.** The method according to any of the preceding claims, wherein the sample is an amplicon prepared from a human or animal sample.

**22.** The method according to claim 21, wherein the sample is an amplicon prepared from sample selected among human blood, urine or tissue.

**23.** The method according to any of the preceding claims, wherein the amplicon is conjugated to a reporter group.

**24.** The method according to any of the preceding claims, wherein the sample is an amplicon prepared from a human or animal sample and the capture oligonucleotide is capable of detecting a SNP.

**25.** The method according to claims 20 or 23, wherein the reporter group is selected among the group consisting of: biotin, digoxigenin, fluorescent groups, dansyl (5-dimethylamino)-1-naphthalenesulfonyl), DOXYL (N-oxyl-4,4-dimethyloxazolidine), PROXYL (N-oxyl-2,2,5,5-tetramethylpyrrolidine), TEMPO (N-oxyl-2,2,6,6-tetramethylpiperidine), dinitrophenyl, acridines, coumarins, Cy3 and Cy5 (trademarks for Biological Detection Systems, Inc.), erythrosine, coumaric acid, umbelliferone, Texas red, rhodamine, tetramethyl rhodamine, Rox, 7-nitrobenzo-2-oxa-1-diazole (NBD), pyrene, fluorescein, Europium, Ruthenium, Samarium, and other rare earth metals, radioisotopic labels, chemiluminescence labels, spin labels, antigens, antibodies, haptens, carrier systems for cell membrane penetration such as: fatty acid residues, steroid moieties (cholesteryl), vitamin A, vitamin D, vitamin E, folic acid peptides for specific receptors, groups for mediating endocytose, epidermal growth factor (EGF), bradykinin, and platelet derived growth factor (PDGF).

**26.** The method according to claim 25, wherein the reporter group is selected from: biotin, fluorescein, Texas Red, rhodamine, dinitrophenyl, digoxigenin. Ruthenium, Europium, Cy5 and Cy3.

**27.** A kit comprising a capture oligonucleotide and a helper probe comprising modified nucleic acid residues.

**28.** The kit of claim 27, wherein the capture oligonucleotide is immobilized on a substrate platform.

**29.** The kit according to claims 27 or 28, wherein the capture oligonucleotide is capable of discriminating between target alleles differing by a SNP.

**30.** The kit according to claims 27 to 29, wherein the capture oligonucleotide or the target is conjugated to a reporter group.

**31.** The kit according to any of claims 27-31, further comprising PCR primers for amplification of the target sequence.

**32.** The kit of claim 31, wherein at least one primer is conjugated to a reporter group.

**33.** Use of an oligonucleotide for enhancing the capture of a target sequence by a capture oligonucleotide, said oligonucleotide comprising modified nucleic acid residues.

**34.** Use of the method according to any of the claims 1 to 26 or the kit according to any of the claims 27 to 32 for genotyping a human or an animal.

**35.** Use of the method according to any of the claims 1 to 26 or the kit according to any of the claims 27 to 32 for a diagnostic assay.

Figure 1A

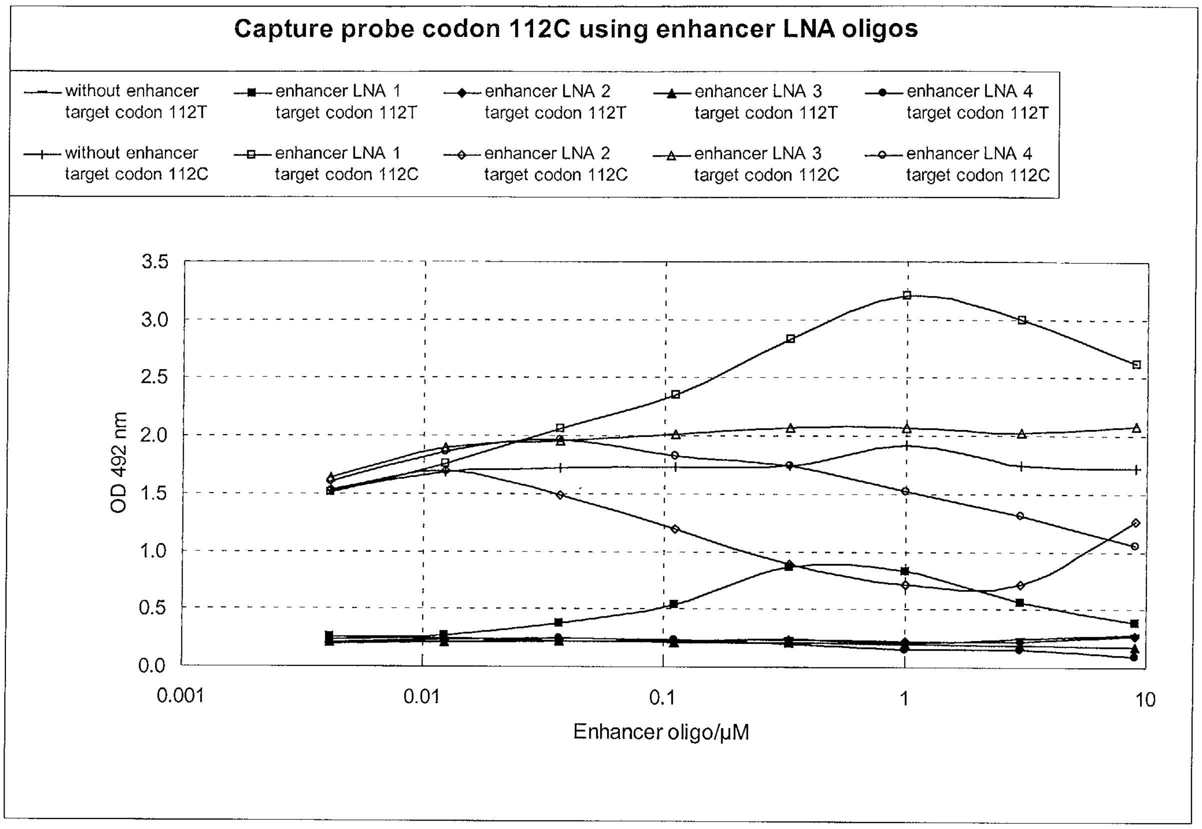
Capture probe codon 112C using enhancer LNA oligos
without enhancer target codon 112T
enhancer LNA 1 target codon 112T
enhancer LNA 2 target codon 112T
enhancer LNA 3 target codon 112T
enhancer LNA 4 target codon 112T
without enhancer target codon 112C
enhancer LNA 1 target codon 112C
enhancer LNA 2 target codon 112C
enhancer LNA 3 target codon 112C
enhancer LNA 4 target codon 112C
OD 492 nm
3.5
3.0
2.5
2.0
1.5
1.0
0.5
0.0
0.001
0.01
0.1
1
10
Enhancer oligo/µM

Figure 1B

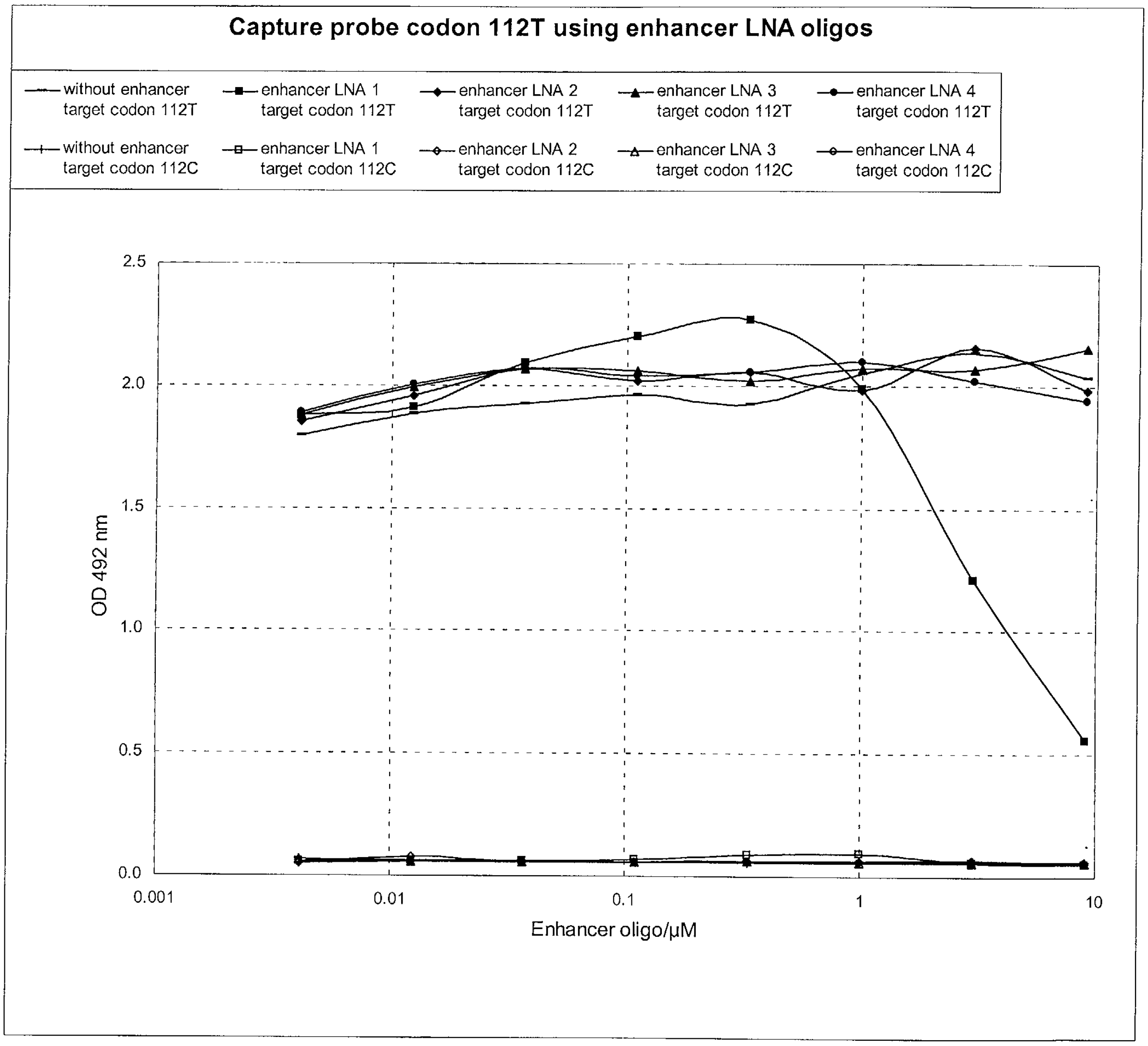
Capture probe codon 112T using enhancer LNA oligos
without enhancer target codon 112T
enhancer LNA 1 target codon 112T
enhancer LNA 2 target codon 112T
enhancer LNA 3 target codon 112T
enhancer LNA 4 target codon 112T
without enhancer target codon 112C
enhancer LNA 1 target codon 112C
enhancer LNA 2 target codon 112C
enhancer LNA 3 target codon 112C
enhancer LNA 4 target codon 112C
2.5
2.0
1.5
1.0
0.5
0.0
OD 492 nm
0.001
0.01
0.1
1
10
Enhancer oligo/µM

Figure 1C

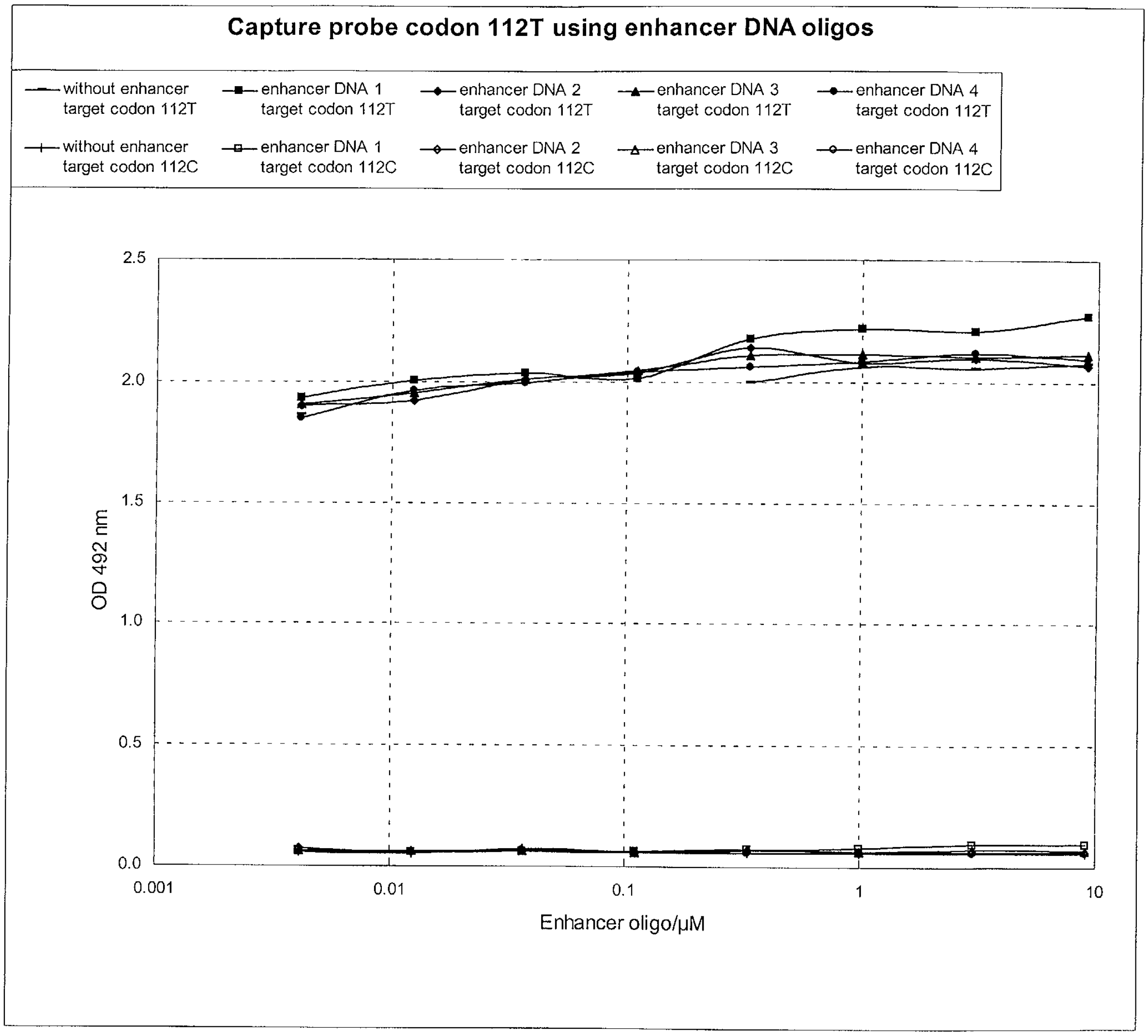
Capture probe codon 112T using enhancer DNA oligos
without enhancer target codon 112T
enhancer DNA 1 target codon 112T
enhancer DNA 2 target codon 112T
enhancer DNA 3 target codon 112T
enhancer DNA 4 target codon 112T
without enhancer target codon 112C
enhancer DNA 1 target codon 112C
enhancer DNA 2 target codon 112C
enhancer DNA 3 target codon 112C
enhancer DNA 4 target codon 112C
OD 492 nm
2.5
2.0
1.5
1.0
0.5
0.0
0.001
0.01
0.1
1
10
Enhancer oligo/µM

Figure 1D

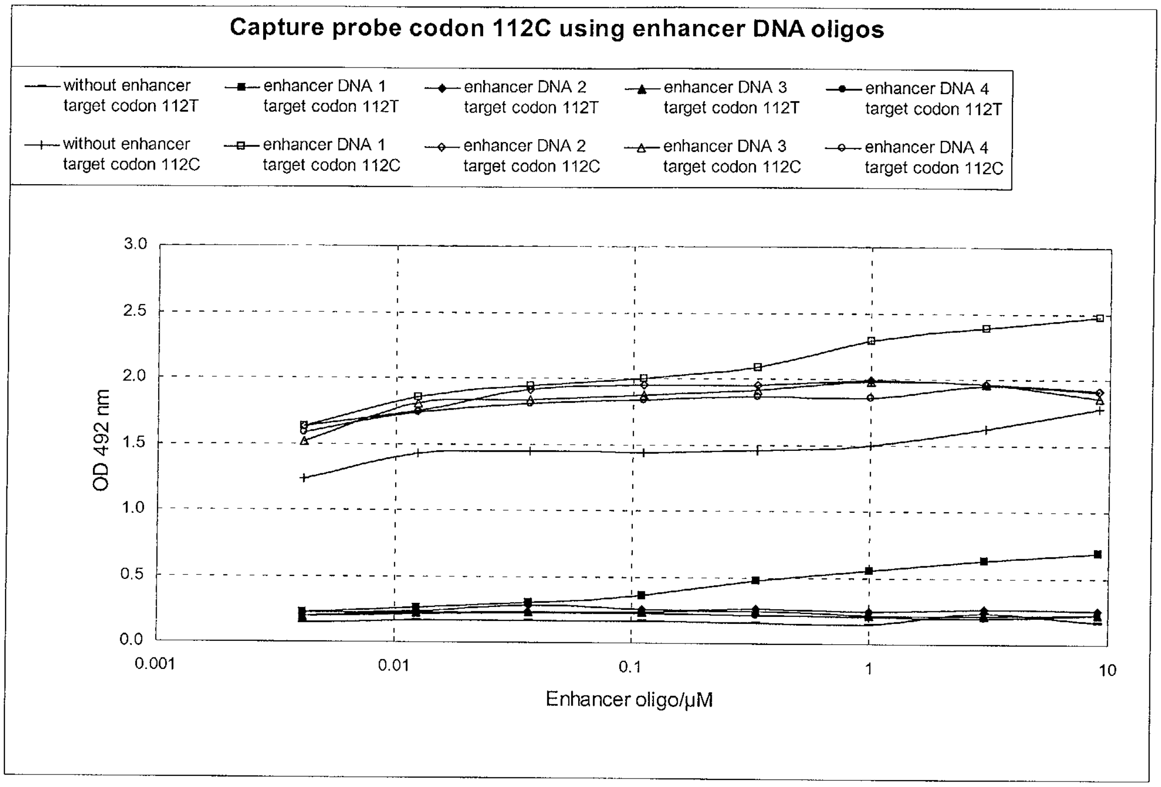
Capture probe codon 112C using enhancer DNA oligos
without enhancer target codon 112T
enhancer DNA 1 target codon 112T
enhancer DNA 2 target codon 112T
enhancer DNA 3 target codon 112T
enhancer DNA 4 target codon 112T
without enhancer target codon 112C
enhancer DNA 1 target codon 112C
enhancer DNA 2 target codon 112C
enhancer DNA 3 target codon 112C
enhancer DNA 4 target codon 112C
OD 492 nm
3.0
2.5
2.0
1.5
1.0
0.5
0.0
0.001
0.01
0.1
1
10
Enhancer oligo/μM

Figure 2

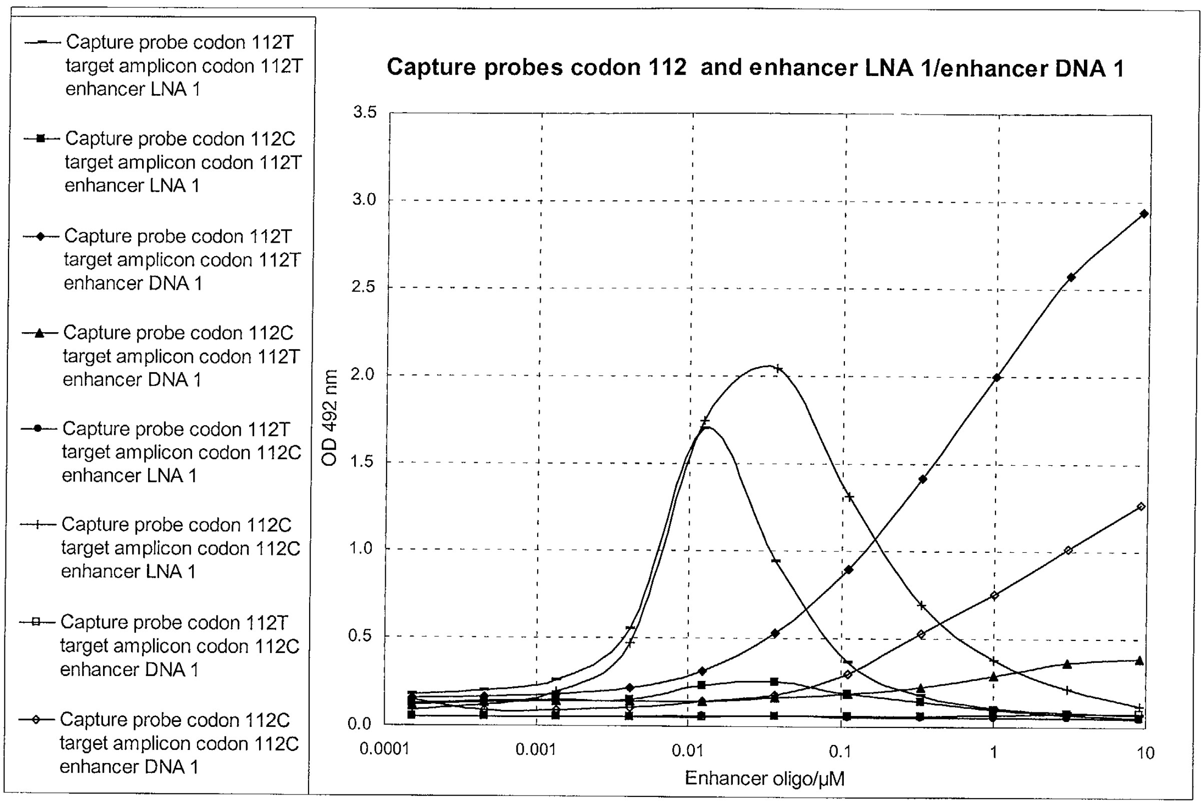
Capture probe codon 112T target amplicon codon 112T enhancer LNA 1
Capture probe codon 112C target amplicon codon 112T enhancer LNA 1
Capture probe codon 112T target amplicon codon 112T enhancer DNA 1
Capture probe codon 112C target amplicon codon 112T enhancer DNA 1
Capture probe codon 112T target amplicon codon 112C enhancer LNA 1
Capture probe codon 112C target amplicon codon 112C enhancer LNA 1
Capture probe codon 112T target amplicon codon 112C enhancer DNA 1
Capture probe codon 112C target amplicon codon 112C enhancer DNA 1
Capture probes codon 112 and enhancer LNA 1/enhancer DNA 1
OD 492 nm
3.5
3.0
2.5
2.0
1.5
1.0
0.5
0.0
0.0001
0.001
0.01
0.1
1
10
Enhancer oligo/μM

Figure 3

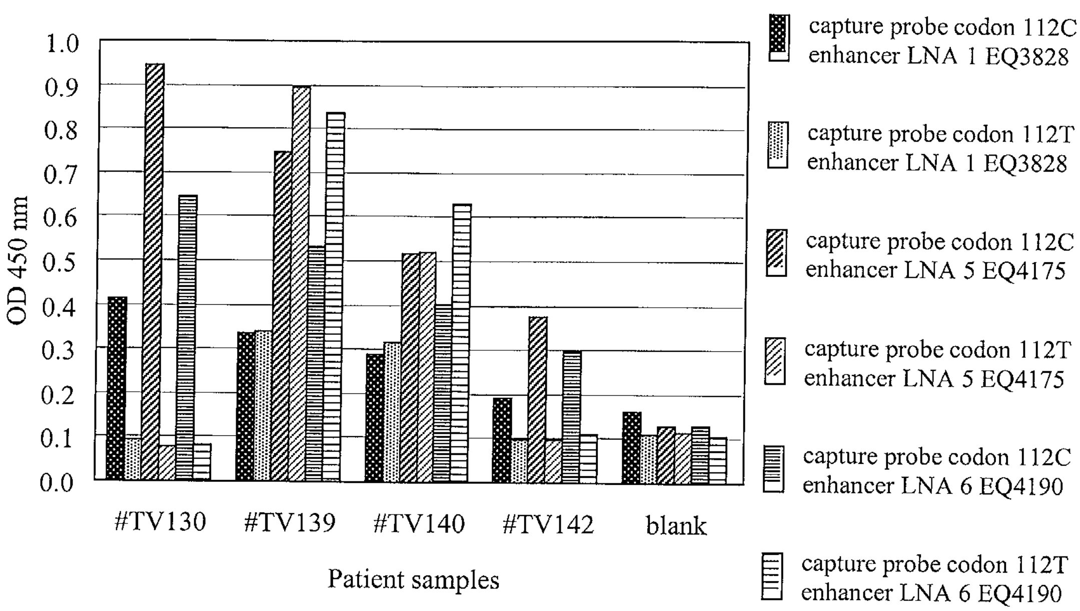
1.0
0.9
0.8
0.7
0.6
0.5
0.4
0.3
0.2
0.1
0.0
OD 450 nm
#TV130
#TV139
#TV140
#TV142
blank
Patient samples
capture probe codon 112C enhancer LNA 1 EQ3828
capture probe codon 112T enhancer LNA 1 EQ3828
capture probe codon 112C enhancer LNA 5 EQ4175
capture probe codon 112T enhancer LNA 5 EQ4175
capture probe codon 112C enhancer LNA 6 EQ4190
capture probe codon 112T enhancer LNA 6 EQ4190